# EUROPEAN PATENT SPECIFICATION

(11) **EP 2 503 992 B1**
(45) Date of publication and mention of the grant of the patent: **20.04.2016**
(21) Application number: 10794865.5
(22) Date of filing: 25.11.2010
(51) Int. Cl.: A61K 9/107, A61K 47/14, A61K 9/00

(54) **FORMULATIONS OF BISPHOSPHONATES AND VITAMIN D SUITABLE FOR INTERMITTENT INTRAMUSCULAR AND SUBCUTANEOUS ADMINISTRATION**
FORMULIERUNGEN AUS BISPHOSPHONATEN UND VITAMIN D FÜR INTERMITTIERENDE INTRAMUSKULÄRE UND SUBKUTANE VERABREICHUNG
FORMULATIONS DE BISPHOSPHONATES ET DE VITAMINE D SE PRÊTANT À UNE ADMINISTRATION INTERMITTENTE PAR VOIE INTRAMUSCULAIRE ET SOUS-CUTANÉE

(30) Priority: 26.11.2009 IT MI20092083
(43) Date of publication of application: 03.10.2012
(73) Proprietor: Romano, Paolo, 20146 Milano (IT); Bruzzese, Tiberio, 20146 Milan (IT)
(72) Inventor: BRUZZESE, Tiberio, I-20146 Milano (IT)
(74) Representative: Ferreccio, Rinaldo
(86) International application number: PCT/EP2010/007140
(87) International publication number: WO 2011/063952

(56) References cited:
- WO-A1-2005/044280
- WO-A1-2008/116809
- WO-A2-2007/038428

## Description

The present invention relates to compositions for pharmaceutical use comprising a substance belonging to the class of bisphosphonates (BF) and vitamin D in high concentrations, dissolved in a small volume of an emulsion of lipids and phospholipids in a concentrated aqueous phase, therefore appropriate for intramuscular (im) and subcutaneous (sc) as well as intermittent use, and useful in the treatment of diseases of the skeletal system, thus avoiding the known detrimental effects against gastro-intestinal tract following the oral administration of BF.

Due to the use of a particular vehicle consisting of an emulsion of lipids in high concentration in water, the composition of the invention is able to dissolve large amounts of the two active ingredients, precisely as required for intermittent dosing. In particular, the main advantage of this composition is to lead to high concentrations of bisphosphonate in bone tissue as a consequence of a decreased rate of absorption and therefore of a lower renal excretion of BF, and above all to give high and prompt blood levels of vitamin D which results to be of high therapeutic value. At the same time the composition is well tolerated at the site of injection and free from local and systemic adverse effects, particularly those relative to the renal system, and was shown to be pharmaceutically quite stable over time.

Bisphosphonates are well known drugs, having a structure (I) similar to that of natural pyrophosphate, an endogenous regulator of calcium metabolism (Fleish H et al, Am J Physiol 1962, 203:671; Fleish H et al Science 1969, 165:1262) and are widely used in clinical practice in all diseases of the skeletal system that lead to excessive bone loss. Having the property to bind to the hydroxyapatite crystals and to inhibit the function of osteoclasts, BF are indeed used as drugs for the treatment and prevention of bone loss associated with the osteoclasts-mediated bone resorption, such as Paget's disease of bone, malignant hypercalcemia, bone metastases and osteoporosis (Rosen JD et al, Drugs 1996, 51:537).

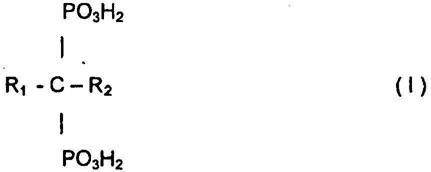

As we shall see, treatment with bisphosphonates has been associated with significant side effects, such as the occurrence of hypocalcemia, which may be particularly intense after parenteral administration and in vitamin D deficient subjects; hypocalcemia may be so severe to become symptomatic, requiring prompt therapies, leading also to life-threatening events, particularly in patients with hypoparathyroidism or cancer. (Maalouf NM et al, Endocrine Pract 2006, 12:48; Altundag O et al, J Clin Oncol 2004, 22:2035; Peter R et al, BMJ 2004, 328:335; Drogue T et al, Indian J Med Sci 2005, 59:542). The use of BF, particularly by the parenteral route, is also associated - as we shall see -to serious renal toxic effects, being able to determine, for example, acute renal failure, nephrotoxic syndrome or focal segmental glomerulosclerosis (Perazzella MA et al, Kidney International 2008, 74:1385; Diel IJ et al, J Support Oncol, 2007, 5:475).

In addition to the above, BF, particularly those containing a nitrogen group, are irritating to biological tissues, such as gastric mucosa, after oral administration (Wallace JL et al, Aliment Pharmacol There 1999 13:1675; Kanatsu K et al, J. Gastroent. and Hepatol. 2004, 19:512).

Among the many BF introduced in human therapy, the most known and used belong to the above structure (I) where R1 and R2 - which can be the same or different from each other - may be hydrogen, halogen (especially chlorine), hydroxyl, amino - or thio-group, variously substituted or unsubstituted, C1-C3 alkyl, optionally substituted with an heterocyclic or homocyclic group, simple or condensed, containing up to two heteroatoms (particularly N), C1-C6 aminoalkyl wherein the amino group can be substituted by a C1-C6 alkyl or is included in a heterocycle.

The best known BF of the formula (I) are particularly represented by clodronate, alendronate, etidronate, neridronate, pamidronate, risedronate, zoledronate, ibandronate, incadronate, olpadronate, tiludronate and others (Ezra A et al, Advan Drug Deliv Reviews 2000, 42:175).

Below, the generic term of BF will indicate all the compounds mentioned above both in the acid form and as salts with pharmaceutically and clinically acceptable bases as known to the Expert in the field, more particularly sodium salts with various degree of salification, as well as the possible hydrated and anhydrous forms, any racemes, enantiomers or diastereoisomers and the various amorphous and polymorphous crystalline forms. All BF, in the various forms, appear as solids extremely soluble in water.

The absorption of BF in the systemic circulation after oral administration is extremely low and with great variability among subjects and within the same subject (Hyldstrup L et al, Calcification Tissue Int 1993, 53:297; Mitchell DY et al, Pharmaceutical Research 2001, 18:166).

As mentioned, BF are highly soluble in water, have a strongly polar structure and a strong negative charge at the pH of the small intestine, being also able to form insoluble complexes with calcium ions and other bivalent cations in the intestinal lumen: all these features are responsible for their poor oral bioavailability as demonstrated in several animal species and in humans (Fogelman I et al, Clin Endocrinol 1986, 24:57).

Based on the ratio between urinary or plasma concentrations (AUC) after intravenous and oral administration in humans, the oral bioavailability was found to be about 0.7% for alendronate (Gertz B et al, Osteoporosis Int 1993, 53:513), 0.3% for pamidronate (Daley-Yates PT et al, Calcif Tissue Int 1991, 49:433),

3-7% for etidronate (Recker RR, Toxicol Appl Pharmacol 1973, 24:580) and 1-2% for clodronate (Plasker GL et al, Drugs 1994, 47:945). The simultaneous ingestion of food reduces the oral absorption to around zero (Laitinen K et al, Bone 2000, 27:293).

Due to poor absorption, therapy with oral BF requires the administration of much higher doses than potentially needed, the majority of which, i.e. 98-99%, is lost to the therapeutic effect being, however, responsible for the high incidence of serious gastrointestinal disorders (epigastric symptoms, excessive flatulence, diarrhea), as well as esophageal inflammation and ulcerations.

It follows that the daily oral therapy with BF is not recommended at all because it requires additional mandatory precautions such as avoiding supine position after intake, to reduce the esophageal (gastro-intestinal reflux) and gastrointestinal problems, as well as taking medication at fasting, to improve absorption, and with the intake of large amounts of water (Dodwell DJ et al Br.J. Cancer 1990, 61:123; Harinek HI et al, Brit J. Res. Ed 1987, 295:1301; Fitton A et al, Drugs 1991, 41:289); despite all measures, poor gastric tolerability is responsible of a modest patients' compliance to treatment (deGroen PC et al, New EngI. J. Med 1996, 335:1016; Rossini et al, Osteoporosis Int. 2006, 17:914).

As a result of BF poor intestinal absorption and GI tolerability, an extensive use of intravenous therapy has been made, particularly in those cases where very high therapeutic doses were necessary, as in the treatment of bone tumors and Paget's disease. The intravenous route of administration, became particularly popular in clinical practice, because (due to the finding of a high bone tropism and a prolonged residence time in the target tissue of BF) intermittent regimens of administration, with weekly, monthly, quarterly or even longer, i.e. annually intervals, was adopted, obviously using in these cases a dose that is cumulatively equivalent to the sum of the individual daily doses(Sartori L et al, Aging Clin. Exp Res 2003, 15:271;Reginster JY et al, Drugs Aging 2007, 24:351; Eastell R et al, J Clin Endocrinol Metab. 2009, 94:3215). Depending on BF potency and bone tropism, a dose f.i. of approximately 5mg of zoledronic acid every year, a dose of 50mg of neridronate about once a month, a dose of 100-200 mg clodronate, 4-2 times a month, can be administered, as examples of BF with high, medium and moderate activity.

However, also the intravenous route of administration is not free from serious limits, in that, as already stated, it may be more frequently associated with the onset of hypocalcemia, a condition that can lead to life threatening events. Furthermore, quite often, it requires that the administration would be performed through slow infusion (usually of several hours), by diluting the drug in large volumes (i.e. 100-1000 ml), to prevent the achievement of high peak plasma levels, which may result in a too fast urinary excretion and possible kidney damage, also leading to reduced bone tissue levels and reduced efficacy. Moreover, this mode of administration should be performed at hospital or at least by trained personnel and can cause local tolerability problems, with possible phlebitis at the injection site, particularly serious in case of occasional extravasation. All this, of course, does not make this route of administration acceptable and suitable, particularly for all those people suffering from "clinically silent" or chronic conditions, such as osteoporosis or certain forms of cancer requiring long-term therapy.

The intramuscular route of administration, for which much lower volumes must be necessarily used, i.e. around 1 or 2 ml, has proven to be a good option for intermittent bisphosphonate therapy because it allows a more complete and less variable absorption of the drug in the circulatory system than the oral route (Lauren L et al, Pharmacol Toxicol 1991, 69:365) and because intramuscular injections can be easily performed at home. For these reasons BF intramuscular treatment achieved a good diffusion, especially in some countries, representing a large percentage of the total BF prescriptions. Although not yet introduced in therapy, also the subcutaneous route of administration allows a good absorption, showing the same advantages of the intramuscular one (Lauren L et al, Pharmacol Toxicol 1991, 69:365; Roemer-Bécuwe C et al, J of Pain and Symptom Management 2003, 26:843).

The use of these routes of administration (intramuscular and subcutaneous), however, has the same problems of the intravenous one (possible onset of hypocalcemia, achievement of high plasma peaks with possible kidney damage), even worsened by the faster drug absorption (by these routes, the maximum blood concentration is reached just 30 minutes after injection) (Muntoni E et al, J. Chromatogr B Analyte Technol Biomed Life Sci 2004, 5:133) as compared to the administration by slow intravenous infusion. These issues restrict the use of intramuscular and subcutaneous routes to the less potent BF or to lower doses, also preventing the adoption of longer administration intervals.

Intramuscular as well as subcutaneous injections of BP, are also very painful, causing local persistent pain, often referred as unbearable by the patient (Rossini et al, Bone 1999, 24:125).

WO 2005/044280 A1 discloses pharmaceutical formulations comprising a compound of the bisphosphonate class in a stabilizing/dispersing vehicle comprising a pharmaceutically acceptable water emulsion of lipis and/or phospholipids, for parenteral, including intramuscular, administration.

In addition to bisphosphonates, another very important substance in the regulation of bone metabolism is represented by vitamin D.

Vitamin D corresponds to two closely related substances, highly soluble in lipids, practically insoluble in water and biologically inactive, consisting of vitamin D3 (cholecalciferol), found in humans and animals, and vitamin D2 (ergocalciferol), present in the world plant, both having a central role in regulating calcium homeostasis (Brown et al, Am J Physiol 1999, 277:F157). A precursor of vitamin D3 is synthesized in the skin and is converted to vitamin D3 by irradiation with ultraviolet light; sun exposure is the main source of vitamin D, providing up to 90% of the required dose. Alternatively, vitamin D3 is obtained from food, especially fatty fish, dairy products and eggs (Stroud ML et al, Australian Family Physician 2008, 37:1002).

Vitamin D is absorbed in the small intestine and then undergoes an hydroxylation to 25-hydroxy-cholecalciferol (calcifediol), endowed with a moderate "intrinsic" biological activity, and/or to an alternative hydroxylation to 1α,25-dihydroxy-cholecalciferol (calcitriol) or 1α-hydroxy-cholecalciferol (alfacalcidol), in which the substitution at position 1 α gives a complete activation. These biologically very active forms of vitamin D3, hydroxylated in the position 1 α , however, due to their high toxicity in mammals as potential cause of hypercalcemia, cannot be administered in humans in high doses, as it would be required by the intermittent schemes of administration. By contrast the 25-hydroxy-cholecalciferol, which is by about 100 times less potent than calcitriol, but is endowed with intrinsic activity and represents the main storage form of vitamin D in the human body, can be administered on intermittent basis at much higher doses without any toxicity as compared to the more active vitamin D.

The term "vitamin D", as used in this text, refers to any active form of vitamin D, i.e. the 1α,25-dihydroxyvitamin D (1α,25-dihydroxycholecalciferol, calcitriol) or its metabolites or its inactive or partially active precursors, such as 25-hydroxyvitamin D3 (25-hydroxycholecalciferol, calcifediol), vitamin D3 (cholecalciferol), vitamin D2 (ergocalciferol), 1-α-hydroxycholecalciferol (alfacalcidol), 25-hydroxyvitamin D3 3-sulphate, dihydroxytachisterol, doxercalciferol, provitamin D3 (7-dehydrocholesterol) and vitamin D derivatives or analogs such as, for example, falecalcitriol, maxacalcitol and paricalcitol.

The physiological role of vitamin D is to maintain adequate levels of calcium in plasma and extracellular spaces, facilitating the absorption, mobilization and retention of Ca and phosphate ions. However, increasing evidence reveals a direct role of vitamin D on bone mineralization with pharmacological activity. At physiological doses, vitamin D behaves similarly to estrogen and bisphosphonates in inhibiting bone resorption (Suda T et al, J of Cellular Biochemistry 2003, 88:259; Ikeda K, Endocrine Journal 2007, 54:1).

Vitamin D deficiency, characterized by serum levels of 25-hydroxy-vitamin D lower than around 9 ng/mL leads to decreased bone mineralization. Insufficient vitamin D leads to an increase of parathyroid hormone (PTH), which in turn causes an increase in osteoclastic activity and loss of calcium from bone, resulting in worsening of osteoporosis especially in the older person. Prolonged lack of vitamin D, depending on its severity and on concomitant calcium deficiency, is considered as a major cause of progressive bone loss, leading to rickets in children and osteoporosis or osteomalacia in adults and the elderly, and therefore must be treated by administration of vitamin D in a very timely way.

It is also important to note that, due to insufficient sun exposure or a low dietary intake, vitamin D supplementation would be useful in otherwise healthy subjects, and particularly in the elderly person who lives in temperate countries. A high prevalence of subjects with low vitamin D levels (57%) was found in patients hospitalized in general medicine ward, with a mean age of 62 years (Thomas MK et al, N Eng J Med 1998, 338:777) or in the elderly immobilized, in fortress at home and in patients with hip fracture (Lips P, Endocrine Rev 2001, 22:47; Holick MF Am J ClinNutr 1994, 60:619).

Although requirements may vary according to patients characteristics, a dose of 400-1,000 IU or more (i.e. 1,200 IU) per day (or total cumulative units corresponding to intermittent intervals, such 7,000-14,000 IU every 1-2 weeks, 30,000-180,000 IU every 1-6 months, or 360,000 IU per year) is considered to be an optimal vitamin D intake, since it was reported as being able to reduce the risk of hip fractures and non-vertebral fractures (Papadimitropoulos E et al, Endocrine Rev 2002, 23:560; Bischoff-Ferrari HA et al, JAMA 2005, 46:676).

Along with the activity on bone metabolism, vitamin D, at doses substantially higher, has been associated with other biological processes including muscle function and cell proliferation. Available data, in fact, demonstrate the utility of vitamin D in reducing falls (and thus, indirectly, resulting fragility fractures) and in the treatment of cancer, especially kidney tumors (Fujioka T et al, J Urol 1998, 160:247), liver tumors (Dalhoff K et al, Br J Cancer 2003, 89:252),tumors of the prostate (Trump DL et al, Cancer 2006, 106:2136) and the colon (Garland C et al, Lancet 1985, 1:307). While doses of vitamin D necessary for falls prevention lie within the limits of good tolerability, doses of vitamin D and/or of its active metabolites, required to obtain beneficial effects in cancer therapy, up to 10,000 IU and above a day, can frequently determine so marked increases in calcium plasma levels to preclude or highly restrict its use in this indication (Schwartz GG et al, Clin Cancer Res 2005, 11:8680).

Vitamin D is available as an oily solution, to be administered orally or intramuscularly.

WO 2007/038428 A2 discloses vitamin D compositions for the treatment of gastrointestinal disorders associated with chemo- or radiation therapy. Such composition can be administered orally, intravenously, parenterally, rectally, topically, nasally or transdermally.

In man, the absorption of vitamin D after oral administration is good and doses of 100,000 IU allow to maintain serum levels of calcidiol above the value of 80 nmol/l for two months, a level considered optimal for fractures prevention (Bischoff -Ferrari HA et al, Am J Clin Nutr 2006, 84:18).

Conversely, the absorption after intramuscular or subcutaneous administration is delayed (with plasma peaks that can be observed even after weeks), variable and can be very restricte (PM Whyte et al, J Clin Endocrinol. Metabol 1979, 48:906; Romagnoli E , et al, J Clin Endocrinol Metab. 2008, 93:3015), vitamin D remaining unchanged at the injection site.

In conclusion, vitamin D and bisphosphonates exert an independent therapeutic effect, useful in osteoporosis and in other metabolic bone diseases and tumors, but, furthermore, they possess pharmacological properties that make their combined administration extremely beneficial and appropriate for therapeutic or preventative use in different clinical situations.

In particular, the combination of the two substances allow to control an important and opposite side effect of each other. In fact the simultaneous administration of vitamin D may be helpful in reducing the hypocalcemia induced by bisphosphonates, especially frequent in the case of parenteral administration of the high doses allowing longer dosing intervals, while bisphosphonates can reduce the hypercalcemia that can frequently occur in the case of adjuvant therapy with high doses of vitamin D and its analogues for the treatment or prevention of cancer.

Equally important is the ability of the combining therapy with vitamin D and bisphosphonates to improve the clinical response of postmenopausal and secondary osteoporosis, measured by bone mineral density and fracture incidence, compared with therapy with bisphosphonates alone (Koster JC, et al, Eur J Clin Pharmacol 1996, 51:145; Dean et al, BMC Musculoskeletal Disorders 2007, 8:3). With respect to this, it was observed that the 66% of subjects treated with bisphosphonates and not receiving vitamin D supplementation (it was reported that vitamin D is prescribed only in 40% of subjects receiving bisphosphonates) had insufficient levels of circulating vitamin D (Allain TJ et al, Rheumatology 2006, 45:487).

Still, the combination of vitamin D with a bisphosphonate may be useful to counteract the reduction of vitamin D metabolites observed during administration of bisphosphonates (Martinez ME et al, Calcif. Tissue Int. 1997, 61:148).

We agree that a pharmaceutical formulation comprising a bisphosphonate and vitamin D in combination would therefore be very useful in therapy, both to obtain the compensatory therapeutic effect of vitamin D or of bisphosphonates, as described above (and for this the absorption of the two drugs should be contemporaneous), but also to improve the compliance to vitamin D treatment in the case of diseases such as osteoporosis in which treatment or prevention with BF is of very long duration. This formulation should be suitable for the intramuscular or subcutaneous routes of administration that are most convenient for the patients, although on the other hand these may induce with greater frequency and severity the side effects (hypocalcemia, renal failure, hypercalcemia) of bisphosphonates and vitamin D.

Therefore, such a formulation should have a volume suitable for intramuscular or subcutaneous use (0.5 - 5ml) and a kinetic of absorption of the two compounds optimal for the declared purposes; in particular the absorption of vitamin D should be rapid, since the absorption of bisphosphonates (whose possible hypocalcemic effect vitamin D should reduce) is fast, and should be complete so as to ensure the supply of the required vitamin D amount for its own pharmacological activity and synergistic activity with BF. The absorption of BP should, vice versa, be delayed to reduce the risk of hypocalcaemia, the risk of kidney damage and the elimination by this route, while improving the bioavailability in bone.

A pharmaceutical combination of BF and vitamin D has so far been made possible only as an oral formulation (i.e., WO 03/086415), rather than injectable, because the two active ingredients are not soluble in the same solvent, being bisphosphonates highly soluble in water while Vitamin D is only soluble in lipids and practically insoluble in aqueous media. Still remain, in respect of such an oral formulation, all the serious criticisms consequent to the very poor oral absorption of bisphosphonates and their severe irritant effect on the gastrointestinal mucosa.

Indeed, it was recently claimed the combining use of BF with vitamin D, also for parenteral route(WO 2008/116809). The cited Patent Application relates to compositions for parenteral use consisting of bisphosphonates, mainly zoledronic acid (3-6 mg, typically 5 mg) or, hypothetically, other nitrogen containing BP, and vitamin D, essentially cholecalciferol, more precisely reporting that a dose of 300,000-600,000 IU of vitamin D is appropriate for intermittent dosing on an annual basis, which - being an international unit of vitamin D3 (1 IU) equal to 0.025 micrograms, corresponds to a dose of 7.5 - 15 mg. Some specific ingredients of the composition such as polysorbates or other dispersant and tension-active agents such as phospholipids (lecithin, phosphatidylcholine) and/or bile acids, are also described.

From a detailed examination of this Application, however, some drawbacks can be observed that make these formulations unsuitable for our purpose. Firstly it was observed that in all the 20 preparative Examples, the volume of the formulations reaches the standard volume of 100 ml, making it unusable for the intramuscular or subcutaneous administration of our interest. It is also clearly stated that the composition is administered as "infusion" and that this requires at least 15 minutes (Application Examples 21-22), thus confirming the non-randomness of the use of relatively high volumes and anyway unfeasible for our use, which requires reduced volumes and a rapid administration.

It is also confirmed that, as bisphosphonates are hydrophilic and vitamin D lipophilic and practically insoluble in water, this results in relevant formulation difficulties.

In this sense, most of the preparative Examples seem to prove that the true solubilizing agent of vitamin D is ethanol (Examples 1-12) and that the other described ingredients have a prevalent dispersing effect. With regard to this, the presence of particulate matter (Examples 1 and 7) with the use of polysorbate and of turbid dispersions and opalescent solutions (Examples 13-17) with the use of lecithin, if not supported by bile salts (Examples 18-19), are reported. Only Example 20 describes the preparation of a lipid emulsion consisting of soybean oil 3% and lecithin 0.4% with up to a maximum of 600,000 IU of vitamin D per 100 ml (6000 IU / ml).

We have instead found that the use of more concentrated emulsions of lipids and phospholipids in water, was able to overcome all difficulties, leading to a complete solution of both BF and vitamin D3 or other vitamins D, and that this can happen in very small volumes, such as those required for intramuscular or subcutaneous injection, as well as in highly concentrated form, such as those required for the cumulative doses needed in the intermittent administration with long intervals.

More specifically, the composition of the present invention includes a BF in a content ranging from 300 mg to 1 mg, preferably from 100 mg to 2 mg, in which the higher value of 100 mg may be represented by clodronic acid at a dose suitable for a single injection every 1-2 weeks and the lower dose by zoledronic acid suitable for a single administration, quarterly or half-yearly, and vitamin D in doses ranging from 3,000 UI to 360,000 IU, as in general may be required for a single administration in intervals from 1 week to one year, preferably in a more restricted dosage range between 7,000 IU and 90,000 IU, appropriate for a single treatment in periods from 1 week to 3 months.

The concentration values reported in the following specification are to be intended as weight percentages referred to the total volume of the formulation, unless differently indicated. The lipids concentration of the emulsion can vary from 5% to 40% of the total volume, while the phospholipid component can vary from 0.5% to 5%: in all these conditions the two active ingredients are perfectly soluble, resulting an emulsion that can be injected either intramuscularly or subcutaneously as "bolus", i.e. well below a minute of duration. The total volume will be, anyway, somewhere between 0.5 ml and 5.0 ml, preferably between 0.5 ml and 3.5 ml, more preferably between 0.5 ml and 2.0 ml.

It follows therefore that the concentration of vitamin D will never be less than 600 IU / ml, and may even reach the concentration of 720,000 IU/ml.

What makes it more relevant, this particular hydro-lipid composition has allowed to modify some pharmacokinetic parameters pertinent to the absorption of the two active ingredients, that seems to us surprising and particularly advantageous for their use. From in vivo tests, performed in animals, it was shown that the absorption of the vitamin component from the injection site was faster and more complete with respect to pure organic solutions, according to data from the literature (Bille N et al, Nord Vet Med 1976, 1991, 28:496). This aspect was also confirmed through direct comparative tests in the animal, where the absorption of vitamin D dissolved in the emulsion of the invention, i.e. in an aqueous concentrated lipid medium, administered intramuscularly, reaches a plateau within a day compared to a conventional solution in only lipid vehicle, that reached similar blood levels after not less than 7-14 days.

Those figures were clearly reflected by the quantity of residual vitamin D in muscle tissue, following administration of two comparative solutions, confirming a faster release and absorption into the systemic circulation from the concentrated emulsion of the invention.

At the same time it was found that the kinetic of absorption of BF is particularly delayed, which - as above mentioned - allows to improve the ratio of the amount of drug that binds to the target bone tissue as compared to the amount that is eliminated by kidneys, with clear benefits in terms of increased efficacy and reduced renal toxicity.

Another further advantage of the formulation of BF and vitamin D of the present invention, has also been observed in terms of improved local tolerance of the intramuscular injection (reduced pain and tissue damage, absence of inflammation) as compared to traditional aqueous formulations of BF. This effect is probably achieved through a so-called effect of physical "barrier" to the muscle fiber of the concentrated lipid phase, although it was verified that BF remains in the aqueous phase of the emulsion and is not incorporated into the lipid phase, as it happens for instance in liposomal preparations.

Moving to a more detailed examination of the composition of the invention, this includes at least one of the BF mentioned in the introduction, under the formula (I), all used and available in the various forms already mentioned (acids and salts, with pharmaceutically acceptable bases, typically sodium salts, all at various degrees of salification; hydrated or anhydrous forms; the racemes, enantiomers and diastereoisomers, where eligible).

The dose of BF will be depending on their specific potency and, compared to the oral dose, should reflect the proportion of orally bioavailable drug, which is around 1% or low percentage of the oral dose, taking into consideration that the absorption with the intramuscular or subcutaneous formulation under study, will be around 100%.

The dose of BF will furthermore depend, of course, by the frequency of intermittent administration envisaged in the present invention, as a reference 7, 30, 90 times the daily dose, respectively for weekly, monthly or quarterly treatment, and so on.

Typical doses will be tentatively from 100 mg to 200 mg for clodronate (every 1-2 up to 2-4 weeks), 50 mg for neridronate (every 2-4 weeks), 2mg, 4mg, 6 mg for zoledronic acid (every 3, 6, 12 months). In general terms, the BF content will be between 300 mg and 1 mg, preferably between 100 mg and 2 mg.

In a preferred embodiment of the invention, vitamin D is used in the form of non-activated precursor of 25-hydroxyvitamin D, for example in the form of cholecalciferol or ergocalciferol. In the most preferred way vitamin D is used in the form of cholecalciferol (Vitamin D3), in a unit dose within a range between a minimum of 3,000 IU gradually up to 360,000 IU for weekly, monthly or annual administration, as an average of unitary doses containing at least 5000 IU, at least 20,000 IU and at least 60,000 IU to be administered once every week, every month and every thre-six months, preferably 7,000 IU, 30,000 IU and 90,000 IU, respectively, for weekly, monthly and quarterly administration, respectively.

Other vitamins belonging to the D group that can be used are ergocalciferol (vitamin D2), at the same doses, its hydroxylated forms such as 25-hydroxycalciferol (calcifediol, a not fully "activated" form but already with intrinsic activity) or even fully activated forms as the metabolites 1α-hydroxy-and 1α, 25-dihydroxycholecalciferol (alfacalcidol and calcitriol, respectively), these in general to be used at doses 100 times lower.

Also usable are the other known metabolites and derivatives of vitamin D, described above.

All the compositions of the invention based on BF and vitamin D, have a volume strictly between 0.5 and 5.0 ml, preferably between 0.5 and 3.5 ml, more preferably between 0.5 and 2.0 ml, and are reserved exclusively for intramuscular and subcutaneous administration and bolus injection. The slow infusion is totally impractical and is still meaningless in clinical terms. Being injectable, the compositions will necessarily be sterile, having also other properties listed below.

Distinctive feature of these new compositions, is the presence of lipids and phospholipids in high concentration, being the lipid component optimal for an effective solubilization of vitamin D. Lipids and phospholipids make a milky looking emulsion, preferably an oil in water emulsion, in which the lipid component is in the form of microscopic droplets of average size less than 1 micron. Due to their high water solubility and polarity, BF will be dissolved in the aqueous phase of the emulsion and not in the lipid phase, while vitamin D is dissolved in the lipid droplets.

Lipids concentration in the formulation usually varies between 5 and 40%, preferably between 10 and 30%, while the concentration of phospholipids varies between 0.5 and 5.0%, preferably between 1.0 and 3.0%.

Lipids and phospholipids used in the formulations of the invention can be of different origin, i.e. animal and/or vegetable and/or semisynthetic.

The lipids belong to the group of mono-, di- and triglycerides, preferably are triglycerides or a mixture of triglycerides which can be substituted with long or medium chain acyl groups.

Typically they are represented by soybean lipids, if of vegetable origin, and fish oils - also in the form of ethyl esters - if of animal source. Fish oils are preferably enriched with omega-3 components, preferably more than 40-50% or even more. Even olive oil, coconut oil and palm oil and others can be used, as well as a mixture of some of these oils.

All these lipids are particularly concentrated and purified, but in relation to their origin, every lipid species can be used, if appropriate for pharmaceutical use.

In general, phospholipids are selected from the group of phosphatidylethanolamine, phosphatidylserine, phosphatidylinositol and in particular from the group of phosphatidylcholine and are represented, among others, by distearoylphosphatidylcholine, dimyristoylphosphatidylcholine and dimyristoylphosphatidylglycerol, if of synthetic origin; by soy lecithin, whether of vegetable origin; by lecithins of egg yolk (phospholipids from egg yolk), whether of animal origin.

The compositions of formulations for pharmaceutical use may also include one or more pharmaceutically acceptable ingredients and/or adjuvant, recognized by the Expert as suitable for an injectable formulation, that even in this case, must be able to completely solubilize the active ingredients, have an optimal pH, be isotonic, etc.

These substances may belong to the group of sugars, such as glucose, sucrose, lactose or mannose; to the group of polyalcohols such as glycerol, xylitol and others; of preservatives such as benzyl alcohol and parabens; of ionic and non ionic surfactants, such as sodium deoxycholate, sodium glycocholic and other bile salts; of sodium lauryl sulfate, of Tweens and Cremophors; basic, neutral or acid buffers, such as alkaline carbonates or bicarbonates, phosphates or TRIS buffer.

The use of alkaline buffers, or of alkali is particularly suitable to adjust the pH of the emulsion to values compatible with the intramuscular and subcutaneous administration, as an example, between 4 and 8, or very preferably between 5 and 7, thereby neutralizing the high acidity of the solutions of BF, also contributing to a better tolerability at the injection site independently from the protective effect of the lipid phase.

Yet frequent can be the addition of the common local anesthetics, typically lidocaine, for the same purpose of a better local tolerability.

The composition usually also contains antioxidants such as tocopherol, ascorbyl palmitate, butylhydroxyanisole and butylhydroxytoluene, and also stabilizers, co-solvents, etc., as known to the Expert.

The composition of the invention, as formulation for pharmaceutical use, can be produced and presented in different ways.

According to a first method, which is also the preferred, the formulation contains directly BF and vitamin D as desired, dissolved in the concentrated lipids and phospholipids emulsion in water, together with all other relevant excipient and/or adjuvant, and is provided as sterile ready to use preparation for the simultaneous administration, in a single composition, of the two active ingredients. As already mentioned, the formulations are intended for exclusive use as intramuscular and subcutaneous bolus, of doses which are multiples than those suited for the possible daily use and therefore suitable for intermittent administration at long intervals.

According to another method, the component BF is dissolved in an aqueous solution, together with any other eventual ingredients and/or adjuvants, while vitamin D is dissolved in the lipid and phospholipid emulsion, together with any other eventual ingredients and/or adjuvants, and then the two separate solutions, in an appropriate volume and concentration, are combined extemporaneously before administration and then injected, or are injected separately in a sequential manner. In a variant of this method, the aqueous solution of BF is presented as a lyophilized, at the dried state, or in anhydrous state as sterile powder.

No difficulties are related to the dissolution of BF in water, which is the preferred procedure; as easily is the direct dissolution of BF in the lipid emulsion, preferably - but not necessarily - already containing vitamin D dissolved.

As for vitamin D is concerned, it is easily dissolved in the preconceived aqueous emulsion, which is the preferred procedure, already containing, where appropriate, the dissolved BF, or it is dissolved in the lipid component and the solution is then emulsified.

Extemporaneous preparations are easily made in vials-syringes.

In the preferred case the terminal phase of the procedure simply implies the mixing of the sterile aqueous solution of BF with the sterile phospholipid and lipid emulsion of vitamin D.

To prepare the emulsions, even with added vitamin D and/or BF, it is obviously required the use of special equipment, such as, for example, the colloidal mill, a pressure homogenizer, an ultrasonic generator, etc., as it is known to the Expert.

Intermediate and final compositions will be controlled to check the size of oil particles (diameter 1 - 3 microns, preferably less) and the emulsion stability over time, to avoid any separation of the lipid phase (coalescence and creaming effect) with increasing size of the oil particles: this aspect, however, is not a critical issue for the intramuscular/subcutaneous injection, as it is for the intravenous drug administration, and can still be resolved by the Expert in the field, by changing concentrations, pH, saline compositions and other parameters, according to practice.

Controls will of course include other aspects relevant to the stability of the two active ingredients and excipients, the isotonicity and sterility of the compositions.

The pharmaceutical formulation obtained as above described is useful in the prevention and treatment of all bone diseases sensitive to BF and vitamin D, in particular bone loss and/or its increased turnover, as well as in conditions of vitamin D deficiency. Overall, these diseases include osteoporosis of any origin, such as postmenopausal osteoporosis, or induced by steroids or glucocorticoids, male osteoporosis, and that caused by other diseases or idiopathic. Moreover Paget's disease; osteoarthritis; bone loss localized and associated with prosthesis implantation or osteolysis; bone fractures; bone diseases associated with metastasis; incomplete or imperfect ossification; periodontal diseases and tooth loss; hypercalcemia of tumor origin; drug induced hypercalcemia; multiple myeloma and other sensitive cancers. Special indication is also osteopenia and its various forms, also induced by prolonged immobilization and bone metastases.

The formulation of the inventions may also represent an effective method of treatment of all such pathologies.

The following Examples are addressed to better explain the invention compositions and their preparation methods, without having any limiting purpose. Other Examples are destined to evidence the unexpected biological results, as experimentally obtained in animals, and report the effect of the emulsions on the absorption of the active ingredients starting from the composition vehicles, as well as the local and systemic tolerance of compositions.

### Example 1

| Composition: | |
|---|---|
| Disodium clodronate | 100 mg |
| Cholecalciferol (7,000 IU) | 0.175 mg |
| Soybean lipids | 330 mg |
| Egg yolk phospholipids | 39.6 mg |
| Glycerol | 82.5 mg |
| Water for injections, q.b. to | 3.3 ml |

Preparation: a 10% lipid emulsion in water is prepared according to standard methods, in the presence of phospholipids and glycerol. Cholecalciferol and sodium clodronate in sterile powder are added with this order, shaken and distributed in ampoules or vials made with neutral glass, always in a sterile environment. It is to be administered intramuscularly, as an example at a rate of one injection every two weeks.

### Example 2

| Composition: | |
|---|---|
| Vial A: disodium clodronate | 100 mg |
| Water for injections, q.b. to | 2.2 ml |
| Vial B: Cholecalciferol (14,000 IU) | 0.35 mg |
| Soybean lipids | 330 mg |
| Egg yolk phospholipids | 39.6 mg |
| Glycerol | 82.5 mg |
| Water for injections, q.b. to | 1.1 ml |

Preparation: a disodium clodronate solution is prepared, dispensed in vial A and sterilized in an autoclave at 121 °C for 15 minutes. Separately a 30% lipid emulsion in water is prepared, in the presence of phospholipids and glycerol, cholecalciferol is added, it is then solubilized through moderate agitation and distributed in the vial B, always in a sterile environment. The contents of the two vials are pooled extemporaneously into the syringe, immediately before use, and is administered intramuscularly as described in Example 1.

### Example 3

| Composition: | |
|---|---|
| Vial A: disodium clodronate | 100 mg |
| Vial B: Cholecalciferol (14,000 IU) | 0.35 mg |
| Soybean lipids | 330 mg |
| Egg yolk phospholipids | 39.6 mg |
| Glycerol | 82.5 mg |
| Water for injections, q.b. to | 3.3 ml |

Preparation: a 5% aqueous solution of sodium clodronate is prepared, that is sterilized by filtration on Millipore filter of 0.2 micron, dosed in the vial A and evaporated to dryness by lyophilization.

Separately a 10% lipid emulsion in water is prepared, cholecalciferol is added, shaken, and distributed into vial B under sterile conditions. The content of vial B is drawn into the syringe immediately before use, added to vial A, and the solution in the emulsion is taken and administered with a frequency of once every 1-2 weeks, for example subcutaneously.

### Example 4

| Composition: | |
|---|---|
| Vial A: disodium clodronate | 200 mg |
| Lidocaine hydrochloride | 35 mg |
| Water for injections, q.b. to | 2.2 ml |

Vial B: as in Example 2

Preparation: the sodium clodronate and lidocaine solution is dosed in vial A and sterilized in an autoclave at 121 °C for 15 minutes (alternatively, the solution is sterilized by filtration prior to dosing on a Millipore filter of 0.2 microns). Separately a 30% lipid emulsion in water is prepared, cholecalciferol is added, it is solubilized and distributed in the vial B as described in Example 2. The contents of the vials are pooled extemporaneously in a syringe immediately before use and is administered intramuscularly.

### Example 5

| Composition: | |
|---|---|
| Vial A: disodium clodronate | 100 mg |
| Vial B: cholecalciferol (28,000 IU) | 0.70 mg |
| Soybean lipids | 660 mg |
| Egg yolk phospholipids | 39.6 mg |
| Glycerol | 82.5 mg |
| Benzyl alcohol | 35 mg |
| Water for injections, q.b. to | 3.3 ml |

Preparation: sodium clodronate is dosed in the vial A as in Example 3.

Separately a 20% lipid emulsion in water is prepared in the presence of phospholipids and glycerol, benzyl alcohol and cholecalciferol are added, the mixture is shaken, and distributed into the vial B. The contents of the vial B is drawn into the syringe before use, added to vial A, and the emulsion is administered subcutaneously or intramuscularly, with intervals of once every 2-4 weeks.

### Example 6

| Composition: | |
|---|---|
| Vial A: disodium clodronate | 100 mg |

| | |
|---|---|
| Water for injections, q.b. to | 1.0 ml |
| Vial B: cholecalciferol (14,000 IU) | 0.35 mg |
| Soybean lipids | 200 mg |
| Egg yolk phospholipids | 24 mg |
| Glycerol | 50.0 mg |
| Water for injections, q.b. to | 1.0 ml |

Preparation: the sodium clodronate solution is dosed in vial A and sterilized in an autoclave at 121 °C for 15 minutes as in Example 2. Separately a 20% lipid emulsion in water is prepared, in the presence of phospholipids and glycerol, cholecalciferol is added, shaken briefly and is distributed in the vial B. The contents of the vials are pooled extemporaneously into a syringe immediately before use being a total volume of 2 ml and administered subcutaneously or intramuscularly.

### Example 7

| Composition: | |
|---|---|
| Disodium clodronate | 100 mg |
| Cholecalciferol (28,000 IU) | 0.70 mg |
| Lipids of fish oil | 200 mg |
| Soybean phospholipids (lecithins) | 24.0 mg |
| Glycerol | 50.0 mg |
| Alpha-tocopherol acetate | 3.5 mg |
| Water for injections, q.b. to | 2.0 ml |
| Buffer, q.b. to | pH 7. |

Preparation: the emulsion of 10% fish oil (enriched with omega-3 fatty acids, preferably> 50%) in water is prepared using standard methods in the presence of soy lecithins and glycerol. Cholecalciferol, sodium clodronate and alfa-tocopherol are added in this order, shaken, buffered to pH7 and distributed in ampoules of neutral glass vials, always under sterile conditions. It is administered subcutaneously or intramuscularly, as an example, at a rate of one injection every 4 weeks.

### Example 8

| Composition: | |
|---|---|
| Neridronate sodium | 50 mg |
| Cholecalciferol (28,000 IU) | 0.70 mg |
| Soybean lipids | 200 mg |
| Egg yolk phospholipids | 24.0 mg |
| Glycerol | 50.0 mg |
| Butyl-hydroxyanisole | 0.05 mg |
| Water for injections, q.b. to | 2.0 ml |
| Buffer, q.b. to | pH 7 |

Preparation: a 10% lipid emulsion in water is prepared using standard methods in the presence of phospholipids and glycerol. Cholecalciferol, sodium neridronate and butylhydroxyanisole are added with this order, shaken, buffered to pH7 and distributed in neutral glass bottles, always under sterile conditions. It is to be administered intramuscularly or subcutaneously, as an example, at a rate of one injection every 4 weeks.

### Example 9

| Composition: | |
|---|---|
| Vial A: sodium neridronate | 50 mg |
| Water for injections, q.b. to | 1.0 ml |
| Bottle B: cholecalciferol (28,000 IU) | 0.70 mg |
| Soybean lipids | 200 mg |
| Egg yolk phospholipids | 24 mg |
| Glycerol | 50.0 mg |
| Water for injections, q.b. to | 1.0 ml |

Preparation: Sodium neridronate is dosed in vial A and sterilized in an autoclave at 121 °C for 15 minutes. Separately a 20% lipid emulsion in water is prepared, in the presence of phospholipids and glycerol, cholecalciferol is added, shaken briefly and is distributed in the vial B. The contents of the vials are pooled extemporaneously into a syringe immediately before use and the total volume of 2 ml is administered intramuscularly or subcutaneously.

### Example 10

| Composition: | |
|---|---|
| Ibandronate sodium | 3 mg |
| Cholecalciferol (90,000 IU) | 2.25 mg |
| Soybean lipids | 100 mg |
| Egg yolk phospholipids | 12.0 mg |
| Glycerol | 25.0 mg |
| Water for injections, q.b. to | 1.0 ml |

Preparation: A 10% lipid emulsion in water is prepared as usual, in the presence of phospholipids and glycerol. Cholecalciferol and alendronate sodium are added in this order, shaken, and distributed in ampoules of neutral glass, always under sterile conditions. It is administered intramuscularly or subcutaneously, as an example, at a rate of one injection every 1-3 months.

### Example 11

| Composition: | |
|---|---|
| Zoledronate sodium | 2.0 mg |
| Cholecalciferol (180,000 IU) | 4.5 mg |
| of Soybean lipids | 100 mg |
| Egg yolk phospholipids | 12.0 mg |
| Glycerol | 25.0 mg |
| Water for injections, q.b. to | 1.0 ml |

Preparation: a 10% lipid emulsion in water is prepared as usual, in the presence of phospholipids and glycerol. Cholecalciferol and sodium zoledronate are add in this order, shaken, and distributed in ampoules of neutral glass, always in a sterile environment. It is administered intramuscularly or subcutaneously, as an example, at the rate of one injection every 6-12 months.

### Example 12

One of the procedures of previous Examples is followed, by using for the composition of the invention to be used as intramuscular or subcutaneous injection, the same dose of BF used for intravenous therapy, or - if unknown - the fraction of oral therapeutic dose that according to pharmacokinetic and bioavailability studies is known to be absorbed into the systemic circulation. These doses provide a better therapeutic efficacy and/or of longer duration with respect to intravenous or oral formulations therefore requiring an equal or lower frequency of administration, in this latter case making possible to extend the period of intermittence.

On this basis, the required dose of vitamin D in the composition is then determined, according to multiples of the required daily dose, that is usually between 400 IU and 1,200 IU, preferably around 1,000 IU.

All this, in the knowledge that both active compounds in the compositions for im or sc use of the invention, provide a quantitative absorption of around 100 %, with improved kinetics of absorption.

As an example therefore it will be used 15 mg of pamidronate, 5 mg of risedronate, 2 mg of ibandronate, all solubilized in the described emulsions and in volumes of 0.5-5 ml, with special reference to the lower volumes, preferably around 1 ml.

### Example 13

The procedure as described in Example 12 is followed, by using multiples of the required daily dose of vitamin D, as dictated by the intervals between doses of the intermittent period. In the case of cholecalciferol (vitamin D3), the daily dose usually ranges between 400 IU and 1,200 IU, and is preferably around 1,000 IU. Similar doses are used for its not fully activated metabolic form, 25-hydroxycholecalciferol, and for ergocalciferol (vitamin D2).

Other vitamins D included in the compositions of the invention and consisting of fully activated metabolites, such as 1α-hydroxycholecalciferol and 1α,25-dihydroxycholecalciferol, require correspondingly reduced doses, of around 100 times lower.

### Studies on pharmacokinetics and local tolerance after intramuscular and subcutaneous injection: effect of increasing concentrations of lipids in the emulsion

### EXAMPLE A

Study on the pharmacokinetic of absorption of vitamin D3 after intramuscular administration and evaluation of the residual at the injection site.

### Materials and methods

One hundred Wistar rats were used, weighing approximately 200 g, divided into four groups of 25 animals. The 4 groups received intramuscularly, in the body of the right anterior tibial muscle, in a volume of 0.5 ml/kg, 25 mg/kg of sodium clodronate and 0.125 mg/kg of a mixture of vitamin D3 (cholecalciferol) and [1,2-3H] cholecalciferol (10% by weight; 0.51 Ci / mmol), diluted in a 3% lipid emulsion, corresponding to Example 20 of patent application WO 2008/116809, or in lipid emulsions with increasing concentrations of lipids, as previously described (respectively, 10%, 20%, 30% of soybean lipids, and 1.2% of egg yolk phospholipids).

Five rats from each group were sacrificed at 6 and 24 hours and after 1, 2 and 3 weeks after dosing, for the determination of plasma 25-hydroxycholecalciferol (calcifediol), derived from body metabolism.

In the animals the amount of labeled vitamin D remained in the muscle was determined by measuring the presence of tritium by a spectrometer with liquid scintigraphy after dissection of the injected muscle and homogenization in ice and phosphate buffer. Confirmation of the source of radioactivity, was performed by chromatography of homogenate extracts. Data on vitamin D absorption after intramuscular administration with lipid emulsions at different concentrations, are shown in Table A1, the percentages of the residual amount in the muscle in Table A2.

**TABLE A1. Calcifediol plasma concentrations after intramuscular administration of vitamin D3 (or 10,000 IU/ml) in lipid emulsions with increasing concentrations of lipids**

| Group | 6 hours ng/ml | 24 hours ng/ml | 1 week ng/ml | 2 weeks ng/ml | 3 weeks ng/ml |
|---|---|---|---|---|---|
| 3% Lipid emulsion | 10.5 +/- 4.7 | 9.7 +/- 3.6 | 15.2 +/-2.1 | 28.5 +/- 9.7 . | 36.2 +/- 15.7 |
| 10% Lipid emulsion | 14.4 +/- 17.1 | 24.3+/-3.1 | 36.5 +/-10.5 | 44.6 +/- 15.7 | 52.5 +/- 16.8 |
| 20% Lipid emulsion | 19.1 +/- 18.3 | 29.1 +/- 3.8 | 46.5 +/- 11.2 | 51.6 +/- 15.7 | 44.5 +/- 14.4 |
| 30% Lipid emulsion | 22.5 +/- 15.2 | 35.6 +/- 3.4 | 52.4 +/- 12.5 | 43.7 +/- 11.7 | 38.5 +/- 13.8 |

As compared to the emulsion containing only 3% of lipids, formulations with higher concentration of lipids, resulted in a faster and higher plasma levels of the active metabolite of vitamin D3. After 10% lipid emulsion an increase of plasma concentrations of calcifediol was measured after only 6 hours after injection while with the 3% emulsion an increase in plasma levels was observed only one week after the administration. The described phenomenon was proportional to the percentage of lipid concentration in the emulsions tested. The percentage of radioactivity remaining in the injected muscle up to 3 weeks after intramuscular injection is reported in Table A2.

**TABLE A2. Percentage of the dose present in muscle from 6 hours to 3 weeks, expressed as a percentage of the radioactivity from baseline**

| Group | 6 hours % | 24 hours % | 1 week % | 2 weeks % | 3 weeks % |
|---|---|---|---|---|---|
| 3% Lipid emulsion | 95.6+/- 8.4 | 75.7 +/- 11.1 | 64.5 +/- 10.7 | 50.2 +/- 15.7 | 48.5 +/- 12.7 |
| 10% Lipid emulsion | 67.5+/- 7.5 | 51.9 +/- 10.8 | 41.5+/ 9.6 | 33.3 +/- 12.6 | 25.5+/-10.1 |
| 20% Lipid emulsion | 58.6 +/- 6.4 | 45.8+/- 9.8 | 34.5 +/- 8.7 | 25.2 +/- 11.6 | 16.8+/- 8.8 |
| 30% Lipid emulsion | 45.9+/- 9.3 | 33.6+/- 7.9 | 26.5 +/- 9.7 | 20.2 +/- 12.3 | 8.5 +/- 7.9 |

The chromatographic analysis of extracts of homogenate showed that 99% of the measured radioactivity was due to vitamin D3. At all considered times, the percentages of radioactivity remained always lower after administration of lipid emulsions at concentrations equal to or greater than 10%, compared with the emulsion containing 3% of fat. In particular, at all considered times, the radioactivity measured after 10% lipid emulsion was always one third lower than that of the formulation with 3% of fat. At all times, the decrease of the radioactivity remained in the muscle was found to correlate to the increase in the percentage of lipids in the formulation.

### EXAMPLE B

Pharmacokinetic study, urinary excretion, and bindingto bone tissue, after intramuscular administration of clodronate sodium in rats.

### Materials and methods.

One hundred and twenty 'Wistar' rats were used of about 200g of weight divided into 5 groups of 24 animals each. The first group received in a volume of 1.0 ml/kg, 25 mg/kg of sodium clodronate and 5 uCi/kg (approximately 1 mg) of 14C-clodronate dissolved in saline 0.9%. The second group of animals received the same dose of 14C-clodronate and clodronate and 0.125 mg/kg of vitamin D3 (cholecalciferol) in the same volume of a lipid emulsion at 3%, corresponding to Example 20 of patent application WO 2008/116809 (soybean lipids equal to 3%, soy lecithin at 70% equal to 0.4%). The third, fourth and fifth group received the same doses of sodium clodronate, 14C clodronate and vitamin D3 dissolved in the same volume of fat emulsions with increasing concentrations of lipids, as previously described (respectively, soybean lipids, at 10%, 20% and 30%, and egg yolk phospholipids at 1.2%).

All injections were made into the thigh muscle.

After 5 and 30 minutes, and 1, 2, 6 and 24 hours, animals were sacrificed (3 rats per time point) and blood was drawn for determination of plasma concentrations of clodronate.

After 2 and 24 hours after administration, from the same groups of rats samples of bone (distal femur and hip) were removed to measure radioactivity.

In addition, the urine of periods 0-24h, 24-48 hours and 48-72 hours were collected in metabolic cages, from 3 rats in each of the groups.

Radioactivity in the different samples was counted with a liquid counter scintigraphy and converted to micrograms of clodronate, according to the method described by Lauren et al.(Pharmacology & Toxicology 1991, 69, 356).

The main findings are reported in Table B1 (pharmacokinetic parameters), Table B2 (concentrations in bone tissue) and TABLE B3 (urinary excretion).

TABLE B1. Pharmacokinetic parameters after intramuscular administration of clodronate in lipid emulsions with increasing concentrations of lipids.

| Group | Cmax(µg/ml) | Tmax (min) | AUC 0-inf (hr µg/ml) | Relative Bioavailability (%) |
|---|---|---|---|---|
| Clodronate aqueous solution | 118 +/- 36 | <5 | 52.7 +/- 19 | |
| 3% Lipid emulsion | 115 +/- 27 | <5 | 48.7 /- 16 | 92.40 |
| 10% Lipid emulsion | 95 +/- 14 | 32 +/- 12 | 54.6 +/- 21 | 103.60 |
| 20% Lipid emulsion | 60 +/- 22 | 60 +/- 16 | 51.3+/-21 | 96.77 |
| 30% Lipid emulsion | 43 +/- 16 | 80 +/- 21 | 53.5+/-17 | 101.51 |

The data show a lower maximum concentration of clodronate after intramuscular injection of the emulsion with at least 10% lipids, compared with the aqueous formulation, and a contemporary lengthening of time to peak plasma concentration, indicating a delayed drug absorption from the injection site. This phenomenon is increased with increasing percentage of lipids in the formulation.

The delayed absorption suggests a more gradual urinary excretion with an increased amount of drug available for binding with bone tissue.

Based on calculated AUC values, as compared to the aqueous formulation, the bioavailability of clodronate conveyed in lipid emulsions was complete and not appreciably different within the different lipid concentrations studied.

**TABLE B2. Clodronate concentration in bone (hip and femur) after 2 and 24 hours after administration**

| Group | bone | Time (hour) | µg/g |
|---|---|---|---|
| Clodronate aqueous solution | Hip | 2 | 69.7 +/- 7.6 |
| | | 24 | 73.2 +/- 12.3 |
| | Femur | 2 | 171.9 +/- 14.3 |
| | | 24 | 181.1 +/- 11.8 |
| 3% Lipid emulsion | Hip | 2 | 57.5 +/- 11.4 |
| | | 24 | 66.2 +/- 16.1 |
| | Femur | 2 | 168.9 +/- 20.4 |
| | | 24 | 175.6 +/- 16.2 |
| 10% Lipid emulsion | Hip | 2 | 76.5 +/- 11.4 |
| | | 24 | 81.2 +/- 16.1 |
| | Femur | 2 | 198.2 +/- 18.3 |
| | | 24 | 188.6 +/- 17.8 |
| 20% Lipid emulsion | Hip | 2 | 88.7 /-+ 14.6 |
| | | 24 | 92.4 +/- 12.6 |
| | Femur | 2 | 201.9 +/- 14.6 |
| | | 24 | 195.4 +/- 21.5 |
| 30% Lipid emulsion | Hip | 2 | 99.7 +/-13.9 |
| | | 24 | 101.3 +/- 18.1 |
| | Femur | 2 | 218.7 +/- 26.4 |
| | | 24 | 201.3 +/- 19.1 |

Clodronate concentration in bone (hip and femur) are shown in Table B2. Concentrations in the femur were higher (more than twice) than those found in the hip. In both skeletal sites, after 2 and24 hours, increasing concentrations of clodronate were observed with increased percentage of lipid in the emulsion while no significant differences in measured values between the aqueous solution and the emulsion with low (3%) concentration of lipid where observed.

**TABLE B3. Cumulative percentage of the clodronate dose, measured in urine**

| Group | 0-24 hours (%) | 24-48 hours (%) | 48-72 hours (%) |
|---|---|---|---|
| Clodronate aqueous solution | 65.2 +/- 4.0 | 68.3 +/- 2.2 | 70.2 +/- 3.9 |
| 3% Lipid Emulsion | 66.4 +/- 3.2 | 69.9 +/- 2.8 | 72.1 +/- 2.9 |
| 10% Lipid Emulsion | 52.2 +/- 4.0 | 62.5 +/- 2.8 | 69.7 +/- 2.6 |
| 20% Lipid Emulsion | 45.3 +/- 2.2 | 55.4 +/- 3.1 | 74.3 /- 3.8 |
| 30% Lipid Emulsion | 40.2 +/- 3.9 | 53.2+/-4.6 | 68.9 +/- 1.8 |

Also the cumulative urinary excretion of clodronate in the 72 hours following injection was not significantly different after administration of clodronate dissolved in emulsions with different concentrations of lipids or in aqueous solution (Table B3). However, the percentage of product excreted in urine within the first 24 hours was lower after administration of clodronate in emulsions with lipid concentration higher than 10% compared to the aqueous formulation or to the emulsion with3% of lipids.

### EXAMPLE C

Pharmacokinetic study after subcutaneous administration of sodium neridronate in rats.

### Materials and methods

A study was conducted using the same design as of the above example B, but using a different bisphosphonate, the neridronate (containing an amino group in its structure, belonging to the so-called class of amino-bisphosphonates), a different route, subcutaneous (injection into the skin of the neck), a different vitamin D [vitamin D2 (ergocalciferol)] at a higher concentration. In particular for Group 1 it was used a neridronate formulation in aqueous solution, for Group 2 a lipid emulsion at 3%, corresponding to Example 20 of patent application WO 2008/116809, while groups 3, 4 and 5 were treated with neridronate in lipid emulsions with concentrations of lipids as previously described, soybean lipids respectively at 10%, 20% and 30%. The dose of neridronate administered in each group was 2.5 mg/kg (in a volume of 0.5 ml/kg) mixed with 14C-neridronate to obtain a final radioactivity of 200 nCi/ml. Emulsions of groups 3, 4 and 5 also contained 0.25 mg/ml of vitamin D2 (ergocalciferol), equivalent to 20,000 IU/ml. The main parameters of neridronate pharmacokinetics (mean +/- SD) were as reported in Table C1.

**TABLE C1. Neridronate pharmacokinetic parameters after subcutaneous administration of lipid emulsions with increasing concentrations of lipids.**

| Group | Cmax (µg/ml) | Tmax (min) | AUC 0-inf (hr µg/ml) | Relative Bioavailability % |
|---|---|---|---|---|
| Neridronate aqueous solution | 11.2 +/- 3.6 | <5 | 12.8 +/- 1.9 | |
| 3% Lipid emulsion | 13.4 +/- 2.2 | <5 | 12.7 +/- 1.7 | 99.2 |
| 10% Lipid emulsion | 8.8 +/- 0.9 | 37 +/- 12 | 11.8 +/- 2.3 | 92.1 |
| 20% Lipid emulsion | 6.3 +/- 1.9 | 58 +/- 15 | 13.5 +/- 1.4 | 105.4 |
| 30% Lipid emulsion | 4.7 +/- 2.4 | 85 +/- 18 | 13.4 +/- 2.2 | 104.6 |

Even with the amino-bisphosphonate neridronate and by a different route of administration (subcutaneous) with respect to the intramuscular one, the same effect exerted by the lipids in the formulation was observed. Starting from the 10% lipid emulsion, the peak plasma concentration [C max] was found to decrease significantly according to the increase of lipids concentration and time to peak [T max] increased similarly. The total amount of drug absorbed, that is the drug bioavailability, was similar in all lipid formulations tested and equal to the amount absorbed after administration of the drug in aqueous solution.

### EXAMPLE D

Similar confirmation of the pharmacokinetic results induced by increasing concentrations of lipids in the formulation have been obtained with compositions of:
- neridronate and calcitriol [1α, 25-(OH)2 vitamin D3], for subcutaneous administration.
- ibandronate and cholecalciferol, for intramuscular administration.
- ibandronate and ergocalciferol, for intramuscular administration.

### EXAMPLE E

### Tolerability by intramuscular route

The test evaluates the local damage caused by intramuscular administration of the compositions under examination. To this purpose the following are used: a solution of sodium clodronate and cholecalciferol in 10% lipid emulsion as described in Example 1 (test); a composition with the same active ingredient dissolved in lipid emulsion at 3%, as described in Example 20 of patent application WO 2008/116809 (reference); a solution of 3% sodium clodronate in water (0.9% NaCl) (control).

Three groups of 5 rats each (average weight 232 g +/- 14) were treated by injection into the tibialis muscle with 1 ml/kg of each of the compositions under study. After 24 hours from treatment, animals were sacrificed and the treated muscle was dissected to show the injection site, and any damage was evaluated using the following arbitrary scale: 0 = normal or weak hyperemia; 1 = hyperemia with hemorrhagic area <1 cm; 2 = hyperemia with hemorrhagic area> 1 cm; 3 = hemorrhagic area> 1 cm with hematoma; 4 = diffuse hematoma> 2 cm. Individual values were as follow:
- Test group: 0,1,0,0,1;
- Reference group: 1,1,2,1,1;
- Control group: 2,3,1,2,2.

It follows that the damage induced by the composition of clodronate and cholecalciferol in 10% lipid emulsion is much lower than that caused when in the aqueous phase, as well as in lipid diluted to 3%.

### Example F

Tolerability by intradermal route.

The response to pain induced by local administration is evaluated. Three groups of three rabbits each were shaved in the back and treated, in the left or right region, by intradermal injection with each of the compositions described in Example 5. Pain caused by injected solutions is proportional to the responses of the animal, which reacts to pain by licking the area of injection.

Rabbits are therefore kept under observation for 30 minutes, counting the number of responses to pain.

Result:
- Group test: 1,1,0,2,1;
- Reference group: 2,3,3,2,5;
- Control group: 6,3,5,7,4.

It is therefore clear that the painful stimulus induced by the intradermal injection of the composition of clodronate and cholecalciferol in 10% lipid emulsion is much lower than that caused by the composition in aqueous solution, as well as in lipid diluted to 3%.

### Example G

### Stability of emulsions

To assess the stability of emulsions, three typical formulations according to Examples 1, 7 and 8, were examined at the microscope at the time of preparation and after 2 months, and compared with a commercial emulsion for parenteral nutrition, free of bisphosphonates and vitamin D.

Microscopic examination was conducted at 2,500 magnifications. All emulsions were composed of tiny droplets of an average diameter of 0.3 microns. To verify signs of coalescence, all droplets larger than 1 micron were counted in 40 microscopic fields (70 x 55 microns). All samples were extremely similar, having a number of droplets between 1 and 3 microns equal to approximately 20/40 fields, and being free from droplets larger than 3 microns.

## Claims

1. A composition for pharmaceutical use comprising a substance of the class of bisphosphonates and vitamin D, wherein all components are dissolved in an emulsion comprising lipids and phospholipids in water at concentration of at least 5% and 0.5% w/v respectively, and in a volume suitable for an intramuscular or subcutaneous administration.

2. A composition according to claim 1, wherein said volume is not higher than 5ml, preferably in the range between 0.5ml and 5ml, more preferably between 0.5 ml and 3.5ml, advantageously between 0.5 and 2.0ml.

3. A composition according to any of the preceding claims, wherein the concentration of said lipids in said emulsion is between 5% and 40% w/v, preferably between 10% and 30% w/v.

4. A composition according to any of the preceding claims, wherein the concentration of said phospholipids in said emulsion is between 0.5% and 5% w/v, preferably between 1.0% and 3.0% w/v.

5. A composition according to any of the preceding claims, wherein the content of the substance of the class of bisphosphonates is not lower than 1 mg, preferably between 1 mg and 300 mg and more preferably between 2mg and 100mg.

6. A composition according to any of the preceding claims, wherein the content of vitamin D is not lower than 3,000 IU, preferably between 3,000 IU and 360,000IU, more preferably between 7,000IU and 90,000 IU.

7. A composition according to any of the preceding claims, wherein the concentration of vitamin D is not lower than 600 IU/ml and preferably between 600 IU/ml and 720,000 IU/ml.

8. A composition according to any of the preceding claims, wherein said vitamin D is vitamin D3 (cholecalciferol), vitamin D2 (ergocalciferol), calcifediol, alfacalcidol or calcitriol, or a combination thereof, or a metabolite, precursor, derivative or analogue thereof, preferably vitamin D3 (cholecalciferol).

9. Acomposition according to any of the preceding claims, wherein the substance of the class of bisphosphonates is a compound of general formula PO (OH) 2 - CR1R2 - PO (OH) 2 (I), or a salt thereof, in which R1 and R2 - that may be the same or different from each other - may be hydrogen, halogen (especially chlorine), hydroxyl, amino- or thio-group, variously substituted or unsubstituted, C1-C3 alkyl, optionally substituted by a simple or condensed heterocyclic or homocyclic group, containing up to two heteroatoms (particularly N), C1-C6 aminoalkyl wherein the amino group may be substituted by C1-C6 alkyl or is included in a heterocycle.

10. A composition according to any of the preceding claims, wherein the substance of the class of bisphosphonates includes the acidic form or salts thereof with pharmaceutically acceptable bases , preferably sodium salts with various degrees of salification, as well as any hydrated and anhydrous forms, any racemates, enantiomers or diastereoisomers and various crystalline, amorphous and polymorphous forms.

11. A composition according to any of the preceding claims, wherein the substance of the class of bisphosphonates is represented by clodronate, alendronate, etidronate, neridronate, pamidronate, risedronate, zoledronate, ibandronate, incadronate, olpadronate, tiludronate, or by a combination thereof.

12. A composition according to any of the preceding claims, wherein said lipids are lipids of animal and/or plant and/or semisynthetic origin, selected from the group of mono-, di- and triglycerides, substituted with long or medium chain acyls, and preferably are triglycerides or a mixture of triglycerides.

13. A composition according to any of the preceding claims, wherein said lipids are represented by soy lipids, olive oil, coconut oil, palm oil and others, as well as by a mixture thereof, preferably by soy lipids, if they are of plant origin; by fish oils, also in the form of ethyl esters and enriched in omega-3 components, preferably above 40-50%, if they are of animal origin.

14. A composition according to any of the preceding claims, wherein said phospholipids are phospholipids of animal and/or plant and/or semi-synthetic origin, selected from the group consisting of phosphatidylethanolamine, phosphatidylserine, phosphatidylinositol and phosphatidylcholine, phosphatidylcholine being preferred and being represented by distearoylphosphatidylcholine, dimyristoylphosphatidylcholine and dimyristoylphosphatidil glycerol if of synthetic origin; by soy lecithin, if of plant origin, by egg yolk lecithin (phospholipids from egg yolk), if of animal origin.

15. A pharmaceutical formulation comprising a composition according to any of the preceding claims and comprising further pharmaceutically acceptable excipients and/or adjuvants.

16. A pharmaceutical formulation according to claim 15, wherein said excipients and/or adjuvants belong to the group of sugars, such as glucose, sucrose, lactose or mannose; polyols such as glycerol, xylitol and others; preservatives such as benzyl alcohol and parabens; ionic and nonionic surfactants such as sodium deoxycholate, sodium glycocholate and other bile salts, sodium lauryl sulfate, Tweens and Cremophors; basic, neutral or acidic buffers, such as alkali carbonates or bicarbonates, phosphates or TRIS buffer; antioxidants such as tocopherol, ascorbyl palmitate, butylhydroxyanisole and butylhydroxytoluene; stabilizers, co-solvents, and others.

17. A pharmaceutical formulation according to claim 16, containing a local anesthetic, preferably lidocaine.

18. A pharmaceutical formulation according to any of claims 15-17, wherein said composition contains said substance of the class of bisphosphonates and vitamin D both directly dissolved in the emulsion of lipids and phospholipids, together with said excipients and/or adjuvants, and is sterile so to be ready for administration.

19. A pharmaceutical formulation according to any of claims 15-17, wherein such substance of the class of bisphosphonates is in aqueous solution, together with said excipients and/or adjuvants, while vitamin D is dissolved in the lipid and phospholipid emulsion, together with said excipients and/or adjuvants, said aqueous solution and said lipid and phospholipid emulsion being intended to be combined extemporaneously before administration and injected.

20. A pharmaceutical formulation according to any of claims 15-17, wherein such substance of the class of bisphosphonates is in aqueous solution, together with said excipients and/or adjuvants, while vitamin D is dissolved in the lipid and phospholipid emulsion, together with said excipients and/or adjuvants, said aqueous solution and said lipid and phospholipid emulsion being intended to be injected separately in a sequential manner.

21. A pharmaceutical formulation according to any of claims 15-17, wherein vitamin D is dissolved in the lipid and phospholipid emulsion, together with said excipients and/or adjuvants, while the substance of the class of bisphosphonates is in the dry state through lyophilization or because dosed in the anhydrous state, as a sterile powder.

22. A pharmaceutical formulation according to claim 21, wherein the components are contained in a vial-syringe, and combined and injected extemporaneously.

23. A pharmaceutical formulation for intramuscular or subcutaneous administration according to any of claims 15-22, for use in the prevention and treatment of all bone diseases that are sensitive to BF and vitamin D, such as bone loss and/or its increased turnover, conditions of vitamin D deficiency and sensitive cancers.

24. A pharmaceutical formulation for intramuscular and subcutaneous administration according to any of claims 16-23, for use in the prevention and treatment of osteoporosis of any origin, such as postmenopausal osteoporosis, osteoporosis induced by steroids or glucocorticoids, male osteoporosis, and osteoporosis induced by other diseases or idiopathic osteoporosis; Paget's disease of bone; osteoarthritis; localized bone loss associated with prosthetic implants or osteolysis; bone fractures; bone diseases related to metastasis; incomplete or imperfect ossification; periodontal disease and tooth loss; hypercalcemia of tumor origin; hypocalcemia induced by drug treatments; sensitive cancers; multiple myeloma; osteopoenia and osteopoenia induced by prolonged immobilization and by bone metastases.

## Patentansprüche

1. Zusammensetzung für die pharmazeutische Anwendung, umfassend eine Substanz der Klasse von Bisphosphonaten und Vitamin D, wobei alle Komponenten gelöst sind in einer Emulsion, die Lipide und Phospholipide in Wasser in einer Konzentration von mindestens 5 bzw. 0,5 Gew.-/Vol.-% umfasst, und in einem Volumen, das für eine intramuskuläre oder subkutane Verabreichung geeignet ist.

2. Zusammensetzung nach Anspruch 1, wobei das Volumen nicht größer als 5 ml ist, bevorzugt im Bereich zwischen 0,5 ml und 5 ml, noch bevorzugter zwischen 0,5 ml und 3,5 ml, vorteilhaft zwischen 0,5 und 2,0 ml liegt.

3. Zusammensetzung nach einem der vorhergehenden Ansprüche, wobei die Konzentration der Lipide in der Emulsion zwischen 5 und 40 Gew.-/Vol.-%, bevorzugt zwischen 10 und 30 Gew.-/Vol.-% liegt.

4. Zusammensetzung nach einem der vorhergehenden Ansprüche, wobei die Konzentration der Phospholipide in der Emulsion zwischen 0,5 und 5 Gew.-/Vol.-%, bevorzugt zwischen 1,0 und 3,0 Gew.-/Vol.-% liegt.

5. Zusammensetzung nach einem der vorhergehenden Ansprüche, wobei der Gehalt der Substanz der Klasse von Bisphosphonaten nicht geringer als 1 mg ist, bevorzugt zwischen 1 mg und 300 mg und noch bevorzugter zwischen 2 mg und 100 mg liegt.

6. Zusammensetzung nach einem der vorhergehenden Ansprüche, wobei der Gehalt an Vitamin D nicht geringer als 3.000 IE ist, bevorzugt zwischen 3.000 IE und 360.000 IE, noch bevorzugter zwischen 7.000 IE und 90.000 IE liegt.

7. Zusammensetzung nach einem der vorhergehenden Ansprüche, wobei die Konzentration von Vitamin D nicht geringer als 600 IE/ml ist und bevorzugt zwischen 600 IE/ml und 720.000 IE/ml liegt.

8. Zusammensetzung nach einem der vorhergehenden Ansprüche, wobei das Vitamin D Vitamin D3 (Cholecalciferol), Vitamin D2 (Ergocalciferol), Calcifediol, Alfacalcidol oder Calcitriol oder eine Kombination davon oder ein Metabolit, Vorläufer, Derivat oder Analogon davon, bevorzugt Vitamin D3 (Cholecalciferol) ist.

9. Zusammensetzung nach einem der vorhergehenden Ansprüche, wobei die Substanz der Klasse von Bisphosphonaten eine Verbindung der allgemeinen Formel PO(OH)2-CR1 R2-PO(OH)2 (I) oder ein Salz davon ist, wobei R1 und R2 - die gleich oder verschieden voneinander sein können - Wasserstoff, Halogen (insbesondere Chlor), Hydroxyl, Amino- oder Thiogruppe, verschieden substituiert oder unsubstituiert, C1-C3-Alkyl, wahlweise durch eine einfache oder kondensierte heterocyclische oder homocyclische Gruppe substituiert, die bis zu zwei Heteroatome (insbesondere N) enthält, C1-C6-Aminoalkyl, wobei die Aminogruppe durch C1-C6-Alkyl substituiert sein kann oder in einem Heterocyclus eingeschlossen ist, sein können.

10. Zusammensetzung nach einem der vorhergehenden Ansprüche, wobei die Substanz der Klasse von Bisphosphonaten die saure Form oder Salze davon mit pharmazeutisch akzeptablen Basen, bevorzugt Natriumsalze mit verschiedenen Salzbildungsgraden, sowie irgendwelche hydratisierten und wasserfreien Formen, irgendwelche Racematen, Enantiomere oder Diastereomere und verschiedene kristalline, amorphe und polymorphe Formen umfasst.

11. Zusammensetzung nach einem der vorhergehenden Ansprüche, wobei die Substanz der Klasse von Bisphosphonaten durch Clodronat, Alendronat, Etidronat, Neridronat, Pamidronat, Risedronat, Zoledronat, Ibandronat, Incadronat, Olpadronat, Tiludronat oder durch eine Kombination davon gebildet ist.

12. Zusammensetzung nach einem der vorhergehenden Ansprüche, wobei die Lipide tierischen und/oder pflanzlichen und/oder halbsynthetischen Ursprungs sind, ausgewählt aus der Gruppe bestehend aus Mono-, Di- und Triglyceriden, die mit lang- oder mittelkettigen Acylen substituiert und bevorzugt Triglyceride oder eine Mischung von Triglyceriden sind.

13. Zusammensetzung nach einem der vorhergehenden Ansprüche, wobei die Lipide gebildet sind durch Sojalipide, Olivenöl, Kokosöl, Palmöl und andere sowie durch eine Mischung davon, bevorzugt durch Sojalipide, wenn sie pflanzlichen Ursprungs sind; durch Fischöle, auch in Form von Ethylestern und mit Omega-3-Komponenten angereichert, bevorzugt von über etwa 40-50 %, wenn sie tierischen Ursprung sind.

14. Zusammensetzung nach einem der vorhergehenden Ansprüche, wobei die Phospholipide Phospholipide tierischen und/oder pflanzlichen und/oder halbsynthetischen Ursprungs sind, ausgewählt aus der Gruppe bestehend aus Phosphatidylethanolamin, Phosphatidylserin, Phosphatidylinositol und Phosphatidylcholin, wobei das Phosphatidylcholin bevorzugt wird und gebildet ist durch Distearoylphosphatidylcholin, Dimyristoylphosphatidylcholin und Dimyristoylphosphatidyglycerin, wenn es synthetischen Ursprungs ist; durch Sojalecithin, wenn es pflanzlichen Ursprungs ist, durch Eigelblecithin (Phospholipide aus Eigelb), wenn es tierischen Ursprungs ist.

15. Pharmazeutische Zubereitung umfassend eine Zusammensetzung nach einem der vorhergehenden Ansprüche und umfassend weitere pharmazeutisch akzeptable Träger und/oder Hilfsmittel.

16. Pharmazeutische Zubereitung nach Anspruch 15, wobei die Träger und/oder Hilfsmittel zu der Gruppe von Zuckern, wie Glucose, Saccharose, Lactose oder Mannose; Polyolen wie Glycerin, Xylit und anderen; Konservierungsmitteln wie Benzylalkohol und Parabenen; ionischen und nichtionischen Tensiden wie Natriumdeoxycholat, Natriumglycocholat und anderen Gallensalzen, Natriumlaurylsulfat, Tween- und Cremophor-Arten; basischen, neutralen oder sauren Puffern wie Alkalicarbonaten oder - bicarbonaten, Phosphaten oder TRIS-Puffer; Antioxidantien wie Tocopherol, Ascorbylpalmitat, Butylhydroxyanisol und Butylhydroxytoluol; Stabilisatoren, Co-Lösungsmitteln und anderen gehören.

17. Pharmazeutische Zubereitung nach Anspruch 16 enthaltend Lokalanästhetikum, bevorzugt Lidocain.

18. Pharmazeutische Zubereitung nach einem der Ansprüche 15-17, wobei die Zusammensetzung die Substanz der Klasse von Bisphosphonaten und Vitamin D, beide direkt in der Emulsion von Lipiden und Phospholipiden gelöst, zusammen mit den Trägern und/oder Hilfsmitteln enthält und steril ist, um für die Verabreichung bereit zu sein.

19. Pharmazeutische Zubereitung nach einem der Ansprüche 15-17, wobei die derartige Substanz der Klasse von Bisphosphonaten sich in wässriger Lösung zusammen mit den Trägern und/oder Hilfsmitteln befindet, während Vitamin D in der Lipid- und Phospholipidemulsion zusammen mit den Trägern/Hilfsmitteln gelöst ist, wobei die wässrige Lösung und die Lipid- und Phospholipidemulsion vor der Verabreichung extemporan kombiniert und dann injiziert werden sollen.

20. Pharmazeutische Zubereitung nach einem der Ansprüche 15-17, wobei die derartige Substanz der Klasse von Bisphosphonaten sich in wässriger Lösung zusammen mit den Trägern/Hilfsmitteln und Hilfsmitteln befindet, während Vitamin D in der in der Lipid- und Phospholipidemulsion zusammen mit den Trägern/Hilfsmitteln gelöst ist, wobei die wässrige Lösung und die Lipid- und Phospholipidemulsion getrennt auf sequenzielle Weise injiziert werden sollen.

21. Pharmazeutische Zubereitung nach einem der Ansprüche 15-17, wobei Vitamin D in der Lipid- und Phospholipidemulsion zusammen mit den Trägern und/oder Hilfsmitteln gelöst ist, während die Substanz der Klasse von Bisphosphonaten sich durch Lyophilisierung oder weil es im wasserfreien Zustand als steriles Pulver dosiert worden ist, im trockenen Zustand befindet.

22. Pharmazeutische Zubereitung nach Anspruch 21, wobei die Komponenten in einer Ampullenspritze enthalten und extemporan kombiniert und injiziert werden.

23. Pharmazeutische Zubereitung zur intramuskulären oder subkutanen Verabreichung nach einem der Ansprüche 15-22 zur Verwendung zur Prävention und Behandlung aller Knochenkrankheiten, die auf BF und Vitamin D ansprechen, wie Knochenverlust und/oder erhöhter -umsatz, Zustände von Vitamin D-Mangel und für Vitamin D empfindliche Krebse.

24. Pharmazeutische Zubereitung für die intramuskuläre und subkutane Verabreichung nach einem der Ansprüche 16-23 zur Verwendung zur Prävention und Behandlung von Osteoporose irgendeines Ursprungs wie beispielsweise postmenopausaler Osteoporose, durch Steroide oder Glucocorticoide induzierter Osteoporose, männlicher Osteoporose und durch andere Krankheiten induzierter Osteoporose oder idiopathischer Osteoporose; Paget-Knochenkarzinom; Osteoarthritis; lokalisiertem, mit Protheseimplantaten assoziiertem Knochenverlust oder Osteolyse; Knochenbrüchen; mit Metastasen verbundenen Knochenkrankheiten; unvollständiger oder mangelhafter Ossifikation; parodontaler Krankheit und Zahnverlust; Hyperkalzämie tumorigen Ursprungs; durch Arzneimittelbehandlungen induzierter Hyperkalzämie; empfindlichen Krebsen; multiplen Myelomen; Osteopönie und durch langzeitige Immobilisierung und Knochenmetastasen induzierter Osteopönie.

## Revendications

1. Composition à usage pharmaceutique comprenant une substance de la classe des bisphosphonates et de la vitamine D, dans laquelle tous les composants sont dissous dans une émulsion comprenant des lipides et des phospholipides dans de l'eau à une concentration d'au moins 5 % et 0,5 % en p/v respectivement, et dans un volume se prêtant à une administration par voie intramusculaire ou sous-cutanée.

2. Composition selon la revendication 1, dans laquelle ledit volume n'est pas supérieur à 5 ml, de préférence est dans la plage située entre 0,5 ml et 5 ml, plus préférablement entre 0,5 ml et 3,5 ml, de façon avantageuse entre 0,5 et 2,0 ml.

3. Composition selon l'une quelconque des revendications précédentes, dans laquelle la concentration desdits lipides dans ladite émulsion se situe entre 5 % et 40 % en p/v, de préférence entre 10 % et 30 % en p/v.

4. Composition selon l'une quelconque des revendications précédentes, dans laquelle la concentration desdits phospholipides dans ladite émulsion se situe entre 0,5 % et 5 % en p/v, de préférence entre 1,0 % et 3,0 % en p/v.

5. Composition selon l'une quelconque des revendications précédentes, dans laquelle la teneur de la substance de la classe des bisphosphonates n'est pas inférieure à 1 mg, de préférence est située entre 1 mg et 300 mg et plus préférablement entre 2 mg et 100 mg.

6. Composition selon l'une quelconque des revendications précédentes, dans laquelle la teneur de la vitamine D n'est pas inférieure à 3 000 UI, de préférence est située entre 3 000 UI et 360 000 UI, plus préférablement entre 7 000 UI et 90 000 UI.

7. Composition selon l'une quelconque des revendications précédentes, dans laquelle la concentration de la vitamine D n'est pas inférieure à 600 UI/ml et de préférence est située entre 600 UI/ml et 720 000 UI/ml.

8. Composition selon l'une quelconque des revendications précédentes, dans laquelle ladite vitamine D est de la vitamine D3 (cholécalciférol), de la vitamine D2 (ergocalciférol), du calcifédiol, de l'alfacalcidol ou du calcitriol, ou une combinaison de ceux-ci, ou un métabolite, précurseur, dérivé ou analogue de ceux-ci, de préférence de la vitamine D3 (cholécalciférol).

9. Composition selon l'une quelconque des revendications précédentes, dans laquelle la substance de la classe des bisphosphonates est un composé de formule générale PO (OH) 2 - CR1R2 - PO (OH) 2 (I), ou un sel de celui-ci, formule dans laquelle R1 et R2 - qui peuvent être identiques l'un à l'autre ou différents l'un de l'autre - peuvent être un atome d'hydrogène, un atome d'halogène (en particulier du chlore), un groupe hydroxyle, amino ou thio, diversement substitué ou non substitué, un groupe alkyle en C1 à C3, facultativement substitué par un groupe hétérocyclique ou homocyclique simple ou condensé, contenant un maximum de deux hétéroatomes (en particulier N), un groupe aminoalkyle en C1 à C6 dans lequel le groupe amino peut être substitué par un groupe alkyle en C1 à C6 ou est inclus dans un hétérocycle.

10. Composition selon l'une quelconque des revendications précédentes, dans laquelle la substance de la classe des bisphosphonates inclut la forme acide ou des sels de celle-ci avec des bases pharmaceutiquement acceptables, de préférence des sels de sodium présentant divers degrés de salification, ainsi que toute forme hydratée et anhydre, tout racémate, énantiomère ou diastéréoisomère et diverses formes cristallines, amorphes et polymorphes.

11. Composition selon l'une quelconque des revendications précédentes, dans laquelle la substance de la classe des bisphosphonates est représentée par le clodronate, l'alendronate, l'étidronate, le néridronate, le pamidronate, le risédronate, le zolédronate, l'ibandronate, l'incadronate, l'olpadronate, le tiludronate, ou par une combinaison de ceux-ci.

12. Composition selon l'une quelconque des revendications précédentes, dans laquelle lesdits lipides sont des lipides d'origine animale et/ou végétale et/ou semi-synthétique, choisis dans le groupe constitué des mono-, di- et triglycérides, substitués par des groupes acyles à chaîne longue ou moyenne, et sont de préférence des triglycérides ou un mélange de triglycérides.

13. Composition selon l'une quelconque des revendications précédentes, dans laquelle lesdits lipides sont représentés par les lipides du soja, l'huile d'olive, l'huile de noix de coco, l'huile de palme et autres, ainsi que par un mélange de ceux-ci, de préférence par les lipides du soja, s'ils sont d'origine végétale ; par les huiles de poisson, également sous la forme d'esters d'éthyle et enrichis en composants oméga-3, de préférence au-delà de 40 à 50 %, s'ils sont d'origine animale.

14. Composition selon l'une quelconque des revendications précédentes, dans laquelle lesdits phospholipides sont des phospholipides d'origine animale et/ou végétale et/ou semi-synthétique, choisis dans le groupe constitué de la phosphatidyléthanolamine, de la phosphatidylsérine, du phosphatidylinositol et de la phosphatidylcholine, la phosphatidylcholine étant préférée et étant représentée par la distéaroylphosphatidylcholine, la dimyristoylphosphatidylcholine et le dimyristoylphosphatidylglycérol si elle est d'origine synthétique ; par la lécithine de soja, si elle est d'origine végétale, par la lécithine de jaune d'oeuf (phospholipides de jaune d'oeuf), si elle est d'origine animale.

15. Formulation pharmaceutique comprenant une composition selon l'une quelconque des revendications précédentes et comprenant d'autres excipients et/ou adjuvants pharmaceutiquement acceptables.

16. Formulation pharmaceutique selon la revendication 15, dans laquelle lesdits excipients et/ou adjuvants appartiennent au groupe des sucres, tels que le glucose, le sucrose, le lactose ou le mannose ; des polyols tels que le glycérol, le xylitol et autres ; des agents de conservation tels que l'alcool benzylique et les parabènes ; des tensioactifs ioniques et non ioniques tels que le désoxycholate de sodium, le glycocholate de sodium et autres sels biliaires, le laurylsulfate de sodium, les Tweens et les Cremophors ; des tampons basiques, neutres ou acides, tels que les carbonates ou bicarbonates alcalins, les phosphates ou un tampon TRIS ; des antioxydants tels que le tocophérol, le palmitate d'ascorbyle, le butylhydroxyanisole et le butylhydroxytoluène ; des stabilisants, des co-solvants et autres.

17. Formulation pharmaceutique selon la revendication 16, contenant un anesthésique local, de préférence de la lidocaïne.

18. Formulation pharmaceutique selon l'une quelconque des revendications 15 à 17, dans laquelle ladite composition contient ladite substance de la classe des bisphosphonates et de la vitamine D tous deux directement dissous dans l'émulsion de lipides et de phospholipides, conjointement avec lesdits excipients et/ou adjuvants, et est stérile de façon à être prête à administrer.

19. Formulation pharmaceutique selon l'une quelconque des revendications 15 à 17, dans laquelle une telle substance de la classe des bisphosphonates est en solution aqueuse, conjointement avec lesdits excipients et/ou adjuvants, tandis que la vitamine D est dissoute dans l'émulsion lipidique et phospholipidique, conjointement avec lesdits excipients et/ou adjuvants, ladite solution aqueuse et ladite émulsion lipidique et phospholipidique étant destinées à être combinées de façon extemporanée avant l'administration et injectées.

20. Formulation pharmaceutique selon l'une quelconque des revendications 15 à 17, dans laquelle une telle substance de la classe des bisphosphonates est en solution aqueuse, conjointement avec lesdits excipients et/ou adjuvants, tandis que la vitamine D est dissoute dans l'émulsion lipidique et phospholipidique, conjointement avec lesdits excipients et/ou adjuvants, ladite solution aqueuse et ladite émulsion lipidique et phospholipidique étant destinées à être injectées séparément de manière successive.

21. Formulation pharmaceutique selon l'une quelconque des revendications 15 à 17, dans laquelle la vitamine D est dissoute dans l'émulsion lipidique et phospholipidique, conjointement avec lesdits excipients et/ou adjuvants, tandis que la substance de la classe des bisphosphonates est à l'état sec suite à sa lyophilisation ou en raison de son dosage à l'état anhydre, sous la forme d'une poudre stérile.

22. Formulation pharmaceutique selon la revendication 21, dans laquelle les composants sont contenus dans une fiole-seringue, et combinés et injectés de façon extemporanée.

23. Formulation pharmaceutique se prêtant à une administration par voie intramusculaire ou sous-cutanée selon l'une quelconque des revendications 15 à 22, pour utilisation dans la prévention et le traitement de toutes les maladies osseuses qui sont sensibles aux BF et à la vitamine D, telles que la perte osseuse et/ou le renouvellement osseux accru, les affections liées à une carence en vitamine D et les cancers sensibles.

24. Formulation pharmaceutique pour administration par voie intramusculaire et sous-cutanée selon l'une quelconque des revendications 16 à 23, pour utilisation dans la prévention et le traitement de l'ostéoporose de toute origine, comme l'ostéoporose post-ménopause, l'ostéoporose induite par les stéroïdes ou les glucocorticoïdes, l'ostéoporose masculine et l'ostéoporose induite par d'autres maladies ou l'ostéoporose idiopathique ; la maladie osseuse de Paget ; l'ostéo-arthrite ; la perte osseuse localisée associée aux implants prothétiques ou à l'ostéolyse ; les fractures osseuses ; les maladies osseuses liées à une métastase ; l'ossification incomplète ou imparfaite ; la parodontopathie et la perte de dents ; l'hypercalcémie d'origine tumorale ; l'hypocalcémie induite par les traitements médicamenteux ; les cancers sensibles ; le myélome multiple ; l'ostéopénie et l'ostéopénie induite par une immobilisation prolongée et par des métastases osseuses.
